(19) Europäisches Patentamt
European Patent Office
Office européen des brevets

(11) Veröffentlichungsnummer: **0 176 903**
**A1**

(12) # EUROPÄISCHE PATENTANMELDUNG

(21) Anmeldenummer: **85111997.4**

(22) Anmeldetag: **21.09.85**

(51) Int. Cl.⁴: **C 07 D 279/12**
C 07 D 265/30, A 61 K 31/535
A 61 K 31/54

(30) Priorität: **04.10.84 DE 3436386**

(43) Veröffentlichungstag der Anmeldung:
**09.04.86 Patentblatt 86/15**

(84) Benannte Vertragsstaaten:
**AT BE CH DE FR GB IT LI NL SE**

(71) Anmelder: **Merck Patent Gesellschaft mit beschränkter Haftung**
**Frankfurter Strasse 250**
**D-6100 Darmstadt(DE)**

(72) Erfinder: **Gante, Joachim, Dr.**
**Stormstrasse 4**
**D-6100 Darmstadt 12(DE)**

(72) Erfinder: **Weber, Wolf-Dietrich, Dr.**
**Lindenweg 32**
**D-6107 Reinheim 1(DE)**

(72) Erfinder: **Sombroek, Johannes, Dr.**
**Robert-Koch-Strasse 32**
**D-6100 Darmstadt 13(DE)**

(72) Erfinder: **Schmitges, Claus, Dr.**
**Karolinger Strasse 5**
**D-6114 Gross-Umstadt(DE)**

(72) Erfinder: **Minck, Klaus Otto, Dr.**
**Büchestrasse 8**
**D-6105 Ober-Ramstadt(DE)**

(54) **(Thio)morpholine.**

(57) Neue (Thio)morpholine der Formel I

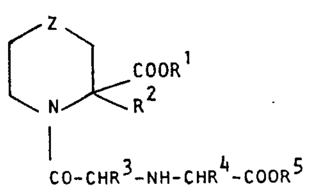

(I)

worin

Z O, S, SO oder $SO_2$,

$R^1$ und $R^5$ gleich oder verschieden sein können und H, Alkyl mit 1 - 4 C-Atomen, Cycloalkyl mit 3 - 7 C-Atomen, Hydroxyalkyl mit 2 - 4 C-Atomen, Alkoxyalkyl mit 2 - 8 C-Atomen oder Benzyl,

$R^2$ H oder Alkyl mit 1 - 6 C-Atomen,

$R^3$ $CH_3$ oder $-(CH_2)_4-NH_2$,

$R^4$ $CH_3$ oder $-CH_2)_2-Ar$ und

Ar unsubstituiertes oder ein- oder mehrfach durch Alkyl mit 1 - 4 C-Atomen, Alkoxy mit 1 - 4 C-Atomen, Cl und/oder CN substituiertes Phenyl bedeuten, sowie deren Salze senken den Blutdruck.

EP 0 176 903 A1

(Thio)morpholine

Die Erfindung betrifft neue (Thio)morpholine der Formel I

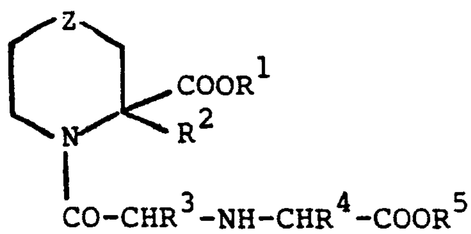

I

worin

Z — O,S, SO oder $SO_2$,

$R^1$ und $R^5$ — gleich oder verschieden sein können und H, Alkyl mit 1 - 4 C-Atomen, Cycloalkyl mit 3 - 7 C-Atomen, Hydroxyalkyl mit 2 - 4 C-Atomen, Alkoxyalkyl mit 2 - 8 C-Atomen oder Benzyl,

$R^2$ — H oder Alkyl mit 1 - 6 C-Atomen,

$R^3$ — $CH_3$ oder $-(CH_2)_4-NH_2$,

$R^4$ — $CH_3$ oder $-(CH_2)_2-Ar$ und

Ar — unsubstituiertes oder ein- oder mehrfach durch Alkyl mit 1 - 4 C-Atomen, Alkoxy mit 1 - 4 C-Atomen, Cl und/oder CN substituiertes Phenyl bedeuten

sowie deren Salze.

Ähnliche Verbindungen sind aus der EP-A-12 401 bekannt, ferner aus der EP-A-48 159, der EP-A-79521 und der US-PS 4 374 829.

Der Erfindung lag die Aufgabe zugrunde,neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze sehr wertvolle Eigenschaften besitzen. Vor allem hemmen sie die Aktivität des "angiotensin converting enzyme" im Humanserum. Diese Wirkung kann z. B. nach der Methode von J. W. Ryan et al., Biochem. J. 167, 501ff (1977) nachgewiesen werden. Bei verschiedenen Formen des erhöhten Blutdrucks zeigen die genannten Verbindungen eine blutdrucksenkende Wirkung. Im einzelnen wirken die Verbindungen blutdrucksenkend bei katheter-tragenden, spontanhypertonen Ratten (Stamm SHR/NIA-Mo/CHB-EMD; Methode vergl. Weeks und Jones, Proc. Soc. Exptl. Biol. Med. 104, 646ff. (1960)) nach intragastraler Gabe der Verbindungen. Weiterhin wird der durch die intravenöse Injektion von Angiotensin I induzierte Blutdruckanstieg bei wachen normotonen Ratten durch intragastrale Vorbehandlung der Tiere mit den Verbindungen vermindert (Methode von Sybertz et al., J. Cardiovascular Pharmacol. 5, 643 ff. (1983)).

Die Verbindungen können als Arzneimittelwirkstoffe in der Human- und Veterinärmedizin eingesetzt werden, insbesondere zur Prophylaxe und zur Behandlung von Herz-, Kreislauf- und Gefäßkrankheiten, vor allem von Hypertonie und Herzinsuffizienz. Außerdem können die Verbindungen zu diagnostischen Zwecken verwendet werden, um z.B. bei Patienten mit Hypertonie den Beitrag des Renin-Angiotensinsystems zur Aufrechterhaltung des pathologischen Zustands zu bestimmen.

In der Formel I haben die Alkyl- und Alkoxygruppen vorzugsweise 1 oder 2, aber auch 3 oder 4, im Fall von $R^2$ auch 5 oder 6 C-Atome. Alkyl bedeutet vorzugsweise Methyl, in zweiter Linie Ethyl, Propyl, Isopropyl, Butyl, Isobutyl, sek.-Butyl oder tert.-Butyl, ferner im Fall von $R^2$ auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl. Alkoxy bedeutet vorzugsweise Methoxy, aber auch Ethoxy, Propoxy, Isopropoxy, Butoxy, Isobutoxy, sek.-Butoxy oder tert.-Butoxy.

Ar bedeutet vorzugsweise Phenyl oder 2,5-Dimethoxyphenyl, ferner bevorzugt o-, m- oder p-Tolyl, o-, m- oder p-Ethylphenyl, o-, m- oder p-Methoxyphenyl, o-, m- oder p-Chlorphenyl, o-, m- oder p-Cyanphenyl, 2,4-, 2,6-, 3,4- oder 3,5-Dimethoxyphenyl, 3,4,5-Trimethoxyphenyl.

Im einzelnen bedeutet Z vorzugsweise S, ferner O, SO oder $SO_2$.

$R^1$ bedeutet vorzugsweise H, Methyl, Ethyl, tert.-Butyl oder Benzyl, ferner Propyl, Isopropyl, Butyl, Isobutyl oder sek.-Butyl.

$R^2$ bedeutet vorzugsweise H oder Methyl, ferner Ethyl, Propyl, Isopropyl, Butyl, Isobutyl, tert.-Butyl, Pentyl, Isopentyl oder Hexyl.

$R^3$ bedeutet vorzugsweise Methyl, ferner 4-Aminobutyl.

$R^4$ bedeutet vorzugsweise 2-Phenylethyl, ferner vorzugsweise Methyl, weiterhin vorzugsweise $-(CH_2)_2-Ar$, worin Ar die oben angegebenen bevorzugten Bedeutungen hat.

$R^5$ ist vorzugsweise H, Methyl, Ethyl, Cyclopentyl, Cyclohexyl, 2-Hydroxyethyl oder 2-Methoxyethyl, ferner Propyl, Isopropyl, Butyl, Isobutyl, sek.-Butyl, tert.-Butyl oder Benzyl.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia und Ib ausgedrückt werden, die der Formel I entsprechen und worin die nicht näher bezeichneten Reste die bei Formel I angegebene Bedeutung haben, worin jedoch

in Ia Z S,
$R^1$ H, Methyl, Ethyl, tert.-Butyl oder Benzyl,
$R^2$ H, Methyl oder Ethyl,
$R^3$ Methyl,
$R^4$ 2-Phenylethyl oder 2-(2,5-Dimethoxyphenyl)-ethyl
$R^5$ H, Methyl oder Ethyl
bedeuten;

in Ib Z S,
$R^1$ H, tert.-Butyl oder Benzyl,
$R^2$ H,
$R^3$ Methyl,
$R^4$ 2-Phenylethyl und
$R^5$ H oder Ethyl
bedeuten.

Die Verbindungen der Formel I besitzen mindestens drei chirale Zentren und können daher bei ihrer Herstellung in verschiedenen Racematen und optisch-aktiven Formen auftreten. Die S,S,S-Formen, (I, Z = O) bzw. die S,S,R-Formen (I, Z = S), die der Stereochemie der natürlichen L-Aminosäuren entsprechen, sind bevorzugt.

Gegenstand der Erfindung ist ferner ein Verfahren zur Herstellung von Verbindungen der Formel I nach Anspruch 1 sowie ihrer Salze, dadurch gekennzeichnet, daß man eine Verbindung der Formel II

$$E - H \qquad II$$

worin

E für den Rest 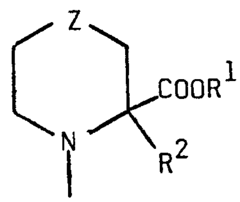 steht und

$R^1$, $R^2$ und Z die in Anspruch 1 angegebene Bedeutung haben

oder eines ihrer reaktionsfähigen Derivate mit einem Dicarbonsäurederivat der Formel III

$$HOOC-CHR^3-NH-CHR^4-COOR^5 \qquad III$$

worin

$R^3$, $R^4$ und $R^5$ die in Anspruch 1 angegebene Bedeutung haben

oder einem seiner reaktionsfähigen Derivate umsetzt,

oder daß man eine Verbindung der Formel IV mit einer Verbindung der Formel V

$$E-CO-CHR^3-X \qquad Y-CHR^4-COOR^5$$

IV V

worin

der eine der Reste X und Y $NH_2$,

der andere X',

X' Cl, Br, J oder eine reaktionsfähig funktionell abgewandelte OH-Gruppe bedeuten und

$R^3$, $R^4$, $R^5$ und E die in Anspruch 1 bzw. oben angegebene Bedeutung haben

umsetzt

oder daß man eine sonst der Formel I entsprechende Verbindung die aber an Stelle eines oder mehrerer H-Atome eine oder mehrere Schutzgruppen und/oder reduzierbare oder durch Wasserstoff ersetzbare Gruppe(n) und/oder C-C-Mehrfachbindung(en) enthält, mit solvolysierenden oder reduzierenden Mitteln behandelt und/oder daß man eine Säure der Formel I ($R^1$ und/oder $R^5$ = H) verestert und/oder einen Ester der Formel I ($R^1$ und/oder $R^5$ sind verschieden von H) in die entsprechende Säure der Formel I ($R^1$ und/oder $R^5$ = H) überführt und/oder ein Thiomorpholin der Formel I (Z = S) zum entsprechenden Sulfoxid der Formel I (Z = SO) oder Sulfon der Formel I ( Z = $SO_2$) oder ein Sulfoxid der Formel I (Z = SO) zum entsprechenden Sulfon der Formel I (Z = $SO_2$) oxydiert und/oder eine Verbindung der Formel I durch Behandeln mit einer Säure oder Base in eines ihrer Salze umwandelt.

Die Verbindungen der Formel I und auch die Ausgangsstoffe zu ihrer Herstellung werden im übrigen nach an sich bekannten Methoden hergestellt, wie sie in der Literatur (z. B. in Standardwerken wie Houben-Weyl, Methoden der Organischen Chemie, Georg-Thieme-Verlag, Stuttgart; ferner EP-A-12401, EP-A-48159, EP-A-79521, US-PS 4 374 829) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Vorzugsweise werden die Verbindungen der Formel I durch

Reaktion von Verbindungen der Formel II mit Dicarbonsäurederivaten der Formel III erhalten. Dabei arbeitet man zweckmäßig nach üblichen Methoden der Peptid-Synthese, wie sie z.B. in Houben-Weyl, l.c., Band 15/II, Seiten 1 bis 806 (1974) beschrieben sind.

Die Reaktion gelingt vorzugsweise in Gegenwart eines Dehydratisierungsmittels, z.B. eines Carbodiimids wie Dicyclohexylcarbodiimid (DCC) oder Dimethylaminopropylethyl-carbodiimid, ferner Diphenylphosphorylazid oder 2-Ethoxy-N-ethoxycarbonyl-1,2-dihydrochinolin, in einem inerten Lösungsmittel, z.B. einem halogenierten Kohlenwasserstoff wie Dichlormethan, einem Ether wie Tetrahydrofuran (THF) oder Dioxan, einem Amid wie Dimethylformamid (DMF) oder Dimethylacetamid, einem Nitril wie Acetonitril, bei Temperaturen zwischen etwa -10 und 40, vorzugsweise zwischen 0 und 30°.

An Stelle von II bzw. III können auch geeignete reaktionsfähige Derivate dieser Stoffe in die Reaktion eingesetzt werden, z.B. solche, in denen reaktive Gruppen intermediär durch Schutzgruppen blockiert sind. Die Aminosäurederivate III können z.B. in Form ihrer aktivierten Ester verwendet werden, die zweckmäßig in situ gebildet werden, z.B. durch Zusatz von 1-Hydroxybenztriazol oder N-Hydroxysuccinimid.

Die Ausgangsstoffe der Formel II sind z.B. erhältlich durch Umsetzung von Carbonsäurederivaten der Formel $X'-CH_2CH_2-Z-CH_2-CR^2-(COOR^1)-X'$ mit Ammoniak oder von Verbindungen der Formel $HZ-CH_2-CR^2(COOR^1)-NH_2$ mit Ethylenoxid oder mit Verbindungen der Formel $X'-CH_2CH_2-X^1$, Alkylderivate der Formel II ($R^2$ = Alkyl) auch durch Alkylierung von Verbindungen der Formel II ($R^2$ = H), worin die NH-Gruppe in üblicher Weise intermediär geschützt wird, Ester (Formel II, $R^1$ = Alkyl oder Benzyl) auch durch Veresterung von Carbonsäuren der Formel II, $R^1$ = H.

Die Ausgangsstoffe der Formel III sind beispielsweise erhältlich durch N-Alkylierung von Verbindungen der Formel $HOOC-CHR^3-NH_2$ mit Verbindungen der Formel V (Y = X') oder von Aminoverbindungen der Formel V (Y = $NH_2$) mit Verbindungen der Formel $HOOC-CHR^3-X'$ nach einer der nachstehend beschriebenen Methoden.

Die Verbindungen der Formel I sind weiterhin herstellbar durch Reaktion von Aminoverbindungen der Formel IV (X = $NH_2$) mit Verbindungen der Formel V ( Y = X') oder von Aminoverbindungen der Formel V (Y = $NH_2$) mit Verbindungen der Formel IV (X = X').

Dabei steht die Gruppe X' bevorzugt für Cl, Br, J oder eine Alkyl- oder Arylsulfonyloxygruppe mit 1 - 10 C-Atomen wie Methan-, Ethan-, Benzol-, p-Toluol- oder 1- oder 2-Naphthalinsulfonyloxy. Die Umsetzung erfolgt zweckmäßig in einem der genannten inerten Lösungsmittel oder Lösungsmittelgemische,z.B. Acetonitril, bei Temperaturen zwischen etwa 0 und 120°, vorzugsweise zwischen etwa 20 und 100°. Der Zusatz einer Base wie Triethylamin oder Pyridin ist zweckmäßig.

Die Verbindungen der Formel I können auch erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die an Stelle einer oder mehrerer freier Amino- und/oder Carboxygruppen entsprechende geschützte Amino- und/oder Carboxygruppen enthalten, vorzugsweise solche, die an Stelle eines H-Atoms, das mit einem N-Atom verbunden ist, eine Aminoschutzgruppe tragen, insbesondere diejenigen der Formel VI

$$E-CO-CH/\overline{(}CH_2)_4-NHQ\overline{7}-NH-CHR^4-COOR^5 \qquad VI$$

worin

Q eine Aminoschutzgruppe bedeutet.

Ferner sind Ausgangsstoffe bevorzugt, die an Stelle des H-Atoms einer Carboxygruppe eine Carboxyschutzgruppe tragen.

Es können auch mehrere - gleiche oder verschiedene - geschützte Amino- und/oder Carboxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acylgruppen, ferner unsubstituierte oder substituierte Aryl- (z. B. 2,4-Dinitrophenyl) oder Aralkylgruppen (z. B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1 - 20, insbesondere 1 - 8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Formyl, Acetyl, Propionyl, Butyryl; Aralkanoyl wie Phenylacetyl; Aroyl wie Benzoyl oder Toluyl; Aryloxyalkanoyl wie Phenoxyacetyl; Alkoxycarbonyl wie Methoxycarbonyl, Ethoxycarbonyl, 2,2,2-Trichlorethoxycarbonyl, tert.-Butoxycarbonyl (BOC), 2-Jodethoxycarbonyl; Aralkyloxycarbonyl wie Benzyloxycarbonyl (CBZ; "Carbobenzoxy"), 4-Methoxybenzyloxycarbonyl, 9-Fluorenylmethoxycarbonyl (FMOC). Bevorzugte Schutzgruppen sind CBZ, FMOC, Benzyl und Acetyl.

Der Ausdruck "Carboxyschutzgruppe" ist ebenfalls allgemein bekannt und bezieht sich auf Gruppen, die geeignet sind, eine Carboxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Carboxyschutzgruppen ist nicht kritisch, da sie nach der gewünschten chemischen Reaktion oder Reaktionsfolge wieder entfernt werden.

Die als Ausgangsstoffe zu verwendenden funktionellen Derivate der Verbindungen der Formel I können nach üblichen Methoden der Aminosäure- und Peptidsynthese hergestellt werden, wie sie z. B. in den genannten Standardwerken und Patentanmeldungen beschrieben sind.

Das In-Freiheit-Setzen der Verbindungen der Formel I aus ihren funktionellen Derivaten gelingt - je nach der benutzten Schutzgruppe - z. B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise die oben angegebenen, ferner z. B. Carbonsäuren wie Essigsäure, auch Alkohole wie Isopropanol oder tert.-Butanol sowie Wasser. Ferner kommen Gemische der vorgenannten

Lösungsmitel in Frage. Trifluoressigsäure wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9 : 1. Die Reaktionstemperaturen für die Spaltung liegen zweckmäßig zwischen etwa 0 und etwa 50°, vorzugsweise arbeitet man zwischen 15 und 30° (Raumtemperatur).

Die BOC-Gruppe kann z. B. bevorzugt mit 40 %iger Trifluoressigsäure in Methylenchlorid oder mit etwa 3 bis 5 n HCl in Dioxan bei 15 - 30° abgespalten werden, die FMOC-Gruppe mit einer etwa 5- bis 20 %igen Lösung von Dimethylamin, Diethylamin oder Piperidin in DMF bei 15 - 30°.

Verbindungen der Formel I sind ferner durch Reduktion entsprechender Verbindungen erhältlich, die aber an Stelle eines oder mehrerer H-Atome eine oder mehrere reduzierbare oder durch Wasserstoff ersetzbare Gruppe(n) und/oder C-C-Mehrfachbindungen enthalten.

Bevorzugte Ausgangsstoffe für die Reduktion entsprechen der Formel VII

$$E\text{-}CO\text{-}G\text{-}COOR^5 \qquad VII$$

worin

G $-CR^3{=}N\text{-}CHR^4\text{-}$, $-CHR^3\text{-}N{=}CR^4\text{-}$, $-CHR^3\text{-}NQ'\text{-}CHR^4\text{-}$, $-CH/\overline{(}CH_2)_4\text{-}NHQ'\overline{7}\text{-}NH\text{-}CHR^4\text{-}$ oder $-CHR^3\text{-}NH\text{-}CH(CH{=}CHAr)\text{-}$ und

Q' eine hydrogenolytisch entfernbare Aminoschutzgruppe bedeuten und

E, Ar und $R^3$ bis $R^5$ die angegebenen Bedeutungen haben.

Hydrogenolytisch entfernbare Schutzgruppen Q' (z. B. CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20 - 30° und 1 - 10 bar durchgeführt. Eine Hydrogenolyse der CBZ-Gruppe gelingt z. B. gut an 5- bis 10 %igem Pd-C in Methanol bei 20 - 30°.

Schiffsche Basen der Formel VII (G = $-CR^3=N-CHR^4-$ bzw. $-CHR^3-N=CR^4-$) werden zweckmäßig hergestellt, indem man entsprechende Carbonylverbindungen der Formeln $E-CO-CO-R^3$ bzw. $R^4-CO-COOR^5$ mit entsprechenden Aminen der Formeln $H_2N-CHR^4-COOR^5$ bzw. $E-CO-CHR^3-NH_2$ umsetzt. Dabei können auch entsprechende Aminole oder Enamine gebildet werden. Alle diese Verbindungen können z. B. mit $NaBH_3CN$ zu Verbindungen der Formel I reduziert werden, zweckmäßig in einem der genannten inerten Lösungsmittel, z. B. einem Alkohol wie Ethanol, einem Nitril wie Acetonitril, in Wasser oder in Gemischen dieser Lösungsmittel bei Temperaturen zwischen etwa 0 und etwa 80°, vorzugsweise zwischen 10 und 40°. Besonders zweckmäßig ist eine Umsetzung in situ, indem man die Carbonylverbindung und das Amin miteinander umsetzt, das Reaktionsprodukt aber nicht isoliert, sondern das Reaktionsgemisch mit dem Reduktionsmittel behandelt. Eine katalytische Hydrierung dieser Ausgangsstoffe, z.B. unter den angegebenen Bedingungen, ist ebenfalls möglich.

Gewünschtenfalls kann eine Säure der Formel I ($R^1$ und/oder $R^5$ = H) nach an sich bekannten Methoden verestert werden, z.B. durch Reaktion mit dem entsprechenden Alkohol in Gegenwart einer Säure oder durch Umsetzung mit einem entsprechenden Diazoalkan.

Weiterhin ist es z. B. möglich, einen Ester der Formel I, worin $R^1$ und/oder $R^5$ von H verschieden sind, in die entsprechende Säure der Formel I ($R^1$ und/oder $R^5$ = H) überzuführen, z. B. mit wässerig-dioxanischer Natronlauge bei Raumtemperatur. tert.-Butylester können selektiv durch Behandeln mit HCl in Dioxan bei etwa 15 - 25° in Carbonsäuren übergeführt werden; dabei werden andere im Molekül vorhandene Alkylestergruppen nicht angegriffen. Benzylester können nach den oben angegebenen Methoden hydrogenolytisch gespalten werden.

Weiterhin kann man in einem Thioether der Formel I die Thioethergruppe zu einer SO-Gruppe oder zu einer $SO_2$-Gruppe oder in einem Sulfoxid der Formel I die SO-Gruppe zu einer $SO_2$-Gruppe oxydieren. Will man die Sulfoxide erhalten, so oxydiert man beispielsweise mit Wasserstoffperoxid, Persäuren wie m-Chlorperbenzoesäure, Cr(VI)-Verbindungen wie Chromsäure, $KMnO_4$, 1-Chlorbenztriazol, Ce(IV)-Verbindungen wie $(NH_4)_2Ce(NO_3)_6$, negativ substituierten aromatischen Diazoniumsalzen wie o- oder p-Nitrophenyldiazoniumchlorid oder elektrolytisch unter verhältnismäßig milden Bedingungen und bei relativ niedrigen Temperaturen (etwa -80 bis +100°). Will man dagegen die Sulfone (aus den Thioethern oder den Sulfoxiden) erhalten, so verwendet man die gleichen Oxydationsmittel unter kräftigeren Bedingungen und/oder im Überschuß sowie in der Regel bei höheren Temperaturen. Bei diesen Umsetzungen können die üblichen inerten Lösungsmittel zugegen oder abwesend sein. Als inerte Lösungsmittel eignen sich beispielsweise Wasser, wässerige Mineralsäuren, wässerige Alkalilaugen, niedere Alkohole wie Methanol oder Ethanol, Ester wie Ethylacetat, Ketone wie Aceton, niedere Carbonsäuren wie Essigsäure, Nitrile wie Acetonitril, Kohlenwasserstoffe wie Benzol, chlorierte Kohlenwasserstoffe wie Chloroform oder $CCl_4$.

Ein bevorzugtes Oxydationsmittel ist 30%iges wässeriges Wasserstoffperoxid. Dieses führt bei Anwendung der berechneten Menge in Lösungsmitteln wie Essigsäure, Aceton, Methanol, Ethanol oder wässeriger Natronlauge bei Temperaturen zwischen -20 und 100° zu den Sulfoxiden, im Überschuß bei höheren Temperaturen, vorzugsweise in Essigsäure oder in einem Gemisch aus Essigsäure und Acetanhydrid, zu den Sulfonen.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z. B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z. B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und disulfonsäure, Laurylschwefelsäure. Salze mit physiologisch nicht unbedenklichen Säuren, z. B. Pikrate, können zur Isolierung und/oder Aufreinigung der Verbindungen der Formel I verwendet werden.

Eine Säure der Formel I kann durch Umsetzung mit einer Base in eines ihrer physiologisch unbedenklichen Metall- bzw. Ammoniumsalze übergeführt werden. Als Salze kommen insbesondere die Natrium-, Kalium-, Magnesium-, Calcium- und Ammoniumsalze in Betracht, ferner substituierte Ammoniumsalze, z. B. die Dimethyl-, Diethyl- oder Diisopropylammonium-, Monoethanol-, Diethanol- und Triethanolammonium-, Cyclohexylammonium-, Dicyclohexylammonium- und Dibenzylethylendiammoniumsalze, weiterhin z. B. Salze mit N-Methyl-D-glucamin oder mit basischen Aminosäuren wie Arginin oder Lysin.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale) oder parenterale Applikation eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen, zur rektalen Anwendung Suppositorien, zur parenteralen Applikation Lösungen, vorzugsweise ölige oder wässerige Lösungen, ferner Suspensionen, Emulsionen oder Implantate. Die neuen Verbindungen können auch lyophilisiert und die erhaltenen Lyophilisate z. B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder

Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z. B. ein oder mehrere Vitamine und/oder Diuretika und/oder Antihypertonika.

Die erfindungsgemäßen Substanzen werden in der Regel in Analogie zu anderen bekannten, im Handel befindlichen Peptiden oder zu Captopril, insbesondere aber in Analogie zu den in der EP-A-12401 beschriebenen Verbindungen wie Enalapril verabreicht, vorzugsweise in Dosierungen zwischen etwa 1 mg und 1 g, insbesondere zwischen 5 mg und 200 mg pro Dosierungseinheit. Die tägliche Dosierung liegt vorzugsweise zwischen etwa 0,07 und 3 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, extrahiert mit Ether oder Dichlormethan, trennt ab, trocknet die organische Phase über Natriumsulfat, filtriert, dampft ein und reinigt durch Chromatographie an Kieselgel und/oder Kristallisation.

Beispiel 1

Zu einem Gemisch von 1,61 g Thiomorpholin-3-carbonsäuremethylester (DL-Form; erhältlich aus 2-Aminoethanthiol und 2,3-Dibrompropionsäuremethylester), 3,39 g N-[1S-Ethoxycarbonyl-3-(2,5-dimethoxyphenyl)-propyl]-L-alanin [erhältlich aus L-Alanin-tert.-butylester und 2-Brom-4-(2,5-dimethoxyphenyl)-buttersäureethylester sowie anschließende Verseifung und Diastereoisomerentrennung], 11,1 ml N-Methylmorpholin, 1,53 g 1-Hydroxybenztriazol und 200 ml $CH_2Cl_2$ tropft man bei 0° unter $N_2$ eine Lösung von 2,06 g DCC in 55 ml $CH_2Cl_2$, rührt 2 Stunden bei 0° und 12 Stunden bei 20° und kühlt auf -70°. Man filtriert, wäscht das Filtrat nacheinander mit 0,5n Salzsäure, gesättigter $NaHCO_3$-Lösung und gesättigter NaCl-Lösung, trocknet, dampft ein, nimmt in Ether auf und erhält 4-[N-(1-Ethoxycarbonyl-3-(2,5-dimethoxyphenyl)-propyl)-alanyl]-thiomorpholin-3-carbonsäuremethylester als öliges Diastereoisomerengemisch, das chromatographisch an Kieselgel in die beiden Formen (S,S,S) und (S,S,R) getrennt werden kann.

Analog erhält man aus den entsprechenden Thiomorpholin-3-carbonsäureestern die S,S,S- und S,S,R-Formen von

4-[N-(1-Ethoxycarbonyl-3-phenylpropyl)-alanyl]-thiomorpholin-3-carbonsäure-methylester
4-[N-(1-Ethoxycarbonyl-3-phenylpropyl)-alanyl]-thiomorpholin-3-carbonsäure-ethylester
4-[N-(1-Ethoxycarbonyl-3-phenylpropyl)-alanyl]-thiomorpholin-3-carbonsäure-n-butylester
4-[N-(1-Ethoxycarbonyl-3-phenylpropyl)-alanyl]-thiomorpholin-3-carbonsäure-tert.-butylester
4-[N-(1-Ethoxycarbonyl-3-phenylpropyl)-alanyl]-thiomorpholin-3-carbonsäure-benzylester

4-[N-(1-Ethoxycarbonyl-3-(2,5-dimethoxyphenyl)-propyl)-alanyl]-thiomorpholin-3-carbonsäureethylester

4-[N-(1-Ethoxycarbonyl-3-(2,5-dimethoxyphenyl)-propyl)-alanyl]-thiomorpholin-3-carbonsäure-tert.-butylester

4-[N-(1-Ethoxycarbonyl-3-(2,5-dimethoxyphenyl)-propyl)-alanyl]-thiomorpholin-3-carbonsäure-benzylester

4-[N-(1-Ethoxycarbonyl-3-(2,5-dimethoxyphenyl)-propyl)-alanyl]-3-methyl-thiomorpholin-3-carbonsäure-methylester

4-[N-(1-Ethoxycarbonyl-3-(2,5-dimethoxyphenyl)-propyl)-alanyl]-3-methyl-thiomorpholin-3-carbonsäure-ethylester

4-[N-(1-Ethoxycarbonyl-3-(2,5-dimethoxyphenyl)-propyl)-alanyl]-3-methyl-thiomorpholin-3-carbonsäure-tert.-butylester

4-[N-(1-Ethoxycarbonyl-3-(2,5-dimethoxyphenyl)-propyl)-alanyl]-3-methyl-thiomorpholin-3-carbonsäure-benzylester

4-[N-(1-Ethoxycarbonyl-3-(3,4-dimethoxyphenyl)-propyl-alanyl]-thiomorpholin-3-carbonsäure-methylester

4-[N-(1-Ethoxycarbonyl-3-(3,4-dimethoxyphenyl)-propyl-alanyl]-thiomorpholin-3-carbonsäure-ethylester

4-[N-(1-Ethoxycarbonyl-3-(3,4-dimethoxyphenyl)-propyl-alanyl]-thiomorpholin-3-carbonsäure-tert.-butylester

4-[N-(1-Ethoxycarbonyl-3-(3,4-dimethoxyphenyl)-propyl-alanyl]-thiomorpholin-3-carbonsäure-benzylester

4-[N-(1-Ethoxycarbonyl-3-(3,4-dimethoxyphenyl)-propyl)-alanyl]-3-methyl-thiomorpholin-3-carbonsäure-methylester

4-[N-(1-Ethoxycarbonyl-3-(3,4-dimethoxyphenyl)-propyl)-alanyl]-3-methyl-thiomorpholin-3-carbonsäure-ethylester

4-[N-(1-Ethoxycarbonyl-3-(3,4-dimethoxyphenyl)-propyl)-alanyl]-3-methyl-thiomorpholin-3-carbonsäure-tert.-butylester

4-[N-(1-Ethoxycarbonyl-3-(3,4-dimethoxyphenyl)-propyl)-alanyl]-3-methyl-thiomorpholin-3-carbonsäure-benzylester

4-[N-(1-Cyclopentoxycarbonyl-3-phenylpropyl)-alanyl]-thiomorpholin-3-carbonsäure-tert.-butylester

4-[N-(1-Cyclohexoxycarbonyl-3-phenylpropyl)-alanyl]-thiomorpholin-3-carbonsäure-tert.-butylester

4-[N-(1-(2-Hydroxyethoxycarbonyl)-3-phenylpropyl)-alanyl]-thiomorpholin-3-carbonsäure-tert.-butylester

4-[N-(1-(2-Methoxyethoxycarbonyl)-3-phenylpropyl)-alanyl]-thiomorpholin-3-carbonsäure-tert.-butylester.

Beispiel 2

Ein Gemisch von 1,75 g Thiomorpholin-3-carbonsäureethylester (R-Form; erhältlich aus L-Cystein und 1,2-Dibromethan), 2,79 g N-(1S-Ethoxycarbonyl-3-phenylpropyl)-L-alanin, 1,23 ml Triethylamin, 1,53 g 1-Hydroxybenztriazol, 2.06 g DCC und 40 ml DMF wird 1 Stunde bei 0° gerührt. Nach üblicher Aufarbeitung erhält man 4-/N-(1-Ethoxycarbonyl-3-phenylpropyl)-alanyl/-thiomorpholin-3-carbonsäure-ethylester, (S,S,R-Form). Hydrochlorid, F. 145-146°.

Analog erhält man aus den entsprechenden Thiomorpholin-3-carbonsäureestern die S,S,R-Formen von

4-/N-(1-Ethoxycarbonyl-3-phenylpropyl)-alanyl/-3-methylthiomorpholin-3-carbonsäure-ethylester

4-/N-(1-Ethoxycarbonyl-3-phenylpropyl)-alanyl/-3-methylthiomorpholin-3-carbonsäure-tert.-butylester

4-/N-(1-Ethoxycarbonyl-3-phenylpropyl)-alanyl/-3-methylthiomorpholin-3-carbonsäure-benzylester

4-/N-(1-Ethoxycarbonyl-3-phenylpropyl-alanyl/-3-ethylthiomorpholin-3-carbonsäure-ethylester

4-/N-(1-Ethoxycarbonyl-3-phenylpropyl-alanyl/-3-ethylthiomorpholin-3-carbonsäure-tert.-butylester

4-/N-(1-Ethoxycarbonyl-3-phenylpropyl-alanyl/-3-ethylthiomorpholin-3-carbonsäure-benzylester

4-/N-(1-Ethoxycarbonyl-3-phenylpropyl)-alanyl/-3-propylthiomorpholin-3-carbonsäure-ethylester

4-/N-(1-Ethoxycarbonyl-3-phenylpropyl)-alanyl/-3-propylthiomorpholin-3-carbonsäure-tert.-butylester

4-/N-(1-Ethoxycarbonyl-3-phenylpropyl)-alanyl/-3-propylthiomorpholin-3-carbonsäure-benzylester

4-/N-(1-Ethoxycarbonyl-3-phenylpropyl)-alanyl/-3- hexylthiomorpholin-3-carbonsäure-ethylester

4-/N-(1-Ethoxycarbonyl-3-phenylpropyl)-alanyl/-3-hexylthiomorpholin-3-carbonsäure-tert.-butylester

4-/N-(1-Ethoxycarbonyl-3-phenylpropyl)-alanyl/-3-hexylthiomorpholin-3-carbonsäure-benzylester

4-[$N^2$-(1-Ethoxycarbonyl-3-phenylpropyl)-lysyl]-thiomorpholin-3-carbonsäure-ethylester

4-[$N^2$-(1-Ethoxycarbonyl-3-phenylpropyl)-lysyl]-thiomorpholin-3-carbonsäure-tert.-butylester

4-[$N^2$-(1-Ethoxycarbonyl-3-phenylpropyl)-lysyl]-thiomorpholin-3-carbonsäure-benzylester

4-[$N^2$-(1-Ethoxycarbonyl-3-phenylpropyl)-lysyl]-3-methylthiomorpholin-3-carbonsäure-ethylester

4-[$N^2$-(1-Ethoxycarbonyl-3-phenylpropyl)-lysyl]-3-methylthiomorpholin-3-carbonsäure-tert.-butylester

4-[$N^2$-(1-Ethoxycarbonyl-3-phenylpropyl)-lysyl]-3-methylthiomorpholin-3-carbonsäure-benzylester.

4-[$N^2$-(1-Ethoxycarbonyl-3-(2,5-dimethoxyphenyl)-propyl)-lysyl]-thiomorpholin-3-carbonsäure-ethylester

4-[$N^2$-(1-Ethoxycarbonyl-3-(2,5-dimethoxyphenyl)-propyl)-lysyl]-thiomorpholin-3-carbonsäure-tert.-butylester

4-[$N^2$-(1-Ethoxycarbonyl-3-(2,5-dimethoxyphenyl)-propyl)-lysyl]-thiomorpholin-3-carbonsäure-benzylester

4-[$N^2$-(1-Ethoxycarbonyl-3-(2,5-dimethoxyphenyl)-propyl)-lysyl]-3-methyl-thiomorpholin-3-carbonsäure-ethylester

4-[$N^2$-(1-Ethoxycarbonyl-3-(2,5-dimethoxyphenyl)-propyl)-lysyl]-3-methyl-thiomorpholin-3-carbonsäure-tert.-butylester

4-[$N^2$-(1-Ethoxycarbonyl-3-(2,5-dimethoxyphenyl)-propyl)-lysyl]-3-methyl-thiomorpholin-3-carbonsäure-benzylester.

## Beispiel 3

Analog Beispiel 1 erhält man aus Morpholin-3-carbonsäureethylester, N-(1S-Carbethoxy-3-phenylpropyl)-L-alaninhydrochlorid, 1-Hydroxybenztriazol, N-Methylmorpholin und N-(3-Dimethylaminopropyl)-N'-ethyl-carbodiimid in $CH_2Cl_2$ 4-[N-(1-Ethoxycarbonyl-3-phenylpropyl)-alanyl]-morpholin-3-carbonsäure-ethylester (S,S,S-Form und S,S,R-Form).

Analog erhält man aus den entsprechenden Morpholin-3-carbonsäureestern die S,S,S- und S,S,R-Formen von:

4-/N-(1-Ethoxycarbonyl-3-phenylpropyl)-alanyl/-morpholin-3-carbonsäure-tert.-butylester

4-/N-(1-Ethoxycarbonyl-3-phenylpropyl)-alanyl/-morpholin-3-carbonsäure-benzylester

4-/N-(1-Ethoxycarbonyl-3-phenylpropyl)-alanyl/-3-methylmorpholin-3-carbonsäure-ethylester

4-/N-(1-Ethoxycarbonyl-3-phenylpropyl)-alanyl/-3-methylmorpholin-3-tert.-butylester

4-/N-(1-Ethoxycarbonyl-3-phenylpropyl)-alanyl/-3-methylmorpholin-3-benzylester.

Beispiel 4

Ein Gemisch von 2,74 g 4-L-Alanyl-thiomorpholin-3-carbonsäure-tert.-butylester (S,R-Form), 2,71 g 2-Brom-4-phenylbuttersäureethylester und 1,01 g Triethylamin in 50 ml Acetonitril wird 12 Stunden gekocht, filtriert und das Filtrat wie üblich aufgearbeitet. Man erhält 4-/N-(1-Ethoxycarbonyl-3-phenylpropyl)-alanyl/-thiomorpholin-3-carbonsäure-tert.-butylester als Diastereoisomerengemisch. Chromatographische Auftrennung liefert die S,S,R-Form und die R,S,R-Form.

Beispiel 5

Man rührt eine Lösung von 4,29 g 4-(2-p-Toluolsulfonyloxypropionyl)-thiomorpholin-3-carbonsäure-tert.-butylester /R-Form; erhältlich aus Thiomorpholin-3-carbonsäure-tert.-butylester (R-Form) und 2-p-Toluolsulfonyloxypropionylchlorid/, 1,01 g Triethylamin und 2,07 g 2-Amino-4-phenylbuttersäureethylester (R-Form) in 15 ml Acetonitril 4 Stunden bei 80°, arbeitet wie üblich auf und erhält 4-/N-(1-Ethoxycarbonyl-3-phenylpropyl)-alanyl/-thiomorpholin-3-carbonsäure-tert.-butylester als Diastereoisomerengemisch, trennbar in S,S,R- und S,R,R-Form.

Beispiel 6

Ein Gemisch von 1 g 4-[$N^2$-(1-Ethoxycarbonyl-3-phenylpropyl)-$N^6$-BOC-lysyl]-thiomorpholin-3-carbonsäure-tert.-butylester [S,S,R-Form; erhältlich aus $N^2$-(1S-Ethoxycarbonyl-3-phenylpropyl)-$N^6$-BOC-L-lysin und R-thiomorpholin-3-carbonsäure-tert.-butylester], 10 ml Dioxan und 20 ml 4n HCl in Dioxan wird 2 Tage bei 20° gerührt. Man dampft ein, nimmt mehrfach mit Toluol und Ether auf, dampft erneut ein, löst in Ethylacetat/Acetonitril und fällt mit Ether das 4-[$N^2$-(1-Ethoxycarbonyl-3-phenylpropyl)-lysyl]-thiomorpholin-3-carbonsäure-dihydrochlorid, S,S,R-Form.

Beispiel 7

Eine Lösung von 217 mg N-(2-Oxopropionyl)-R-thiomorpholin-3-carbonsäure und 207 mg 2-Amino-4-phenylbuttersäureethylester in 5 ml Ethanol wird 1 Stunde bei 20° mit 3 g Molekularsieb Typ 4A gerührt. Man gibt innerhalb 3 Stunden portionsweise 75 mg $NaBH_3CN$ hinzu und reinigt das Produkt durch Adsorption an starkem Kationenaustauscherharz und Elution mit 2% Pyridin in Wasser. Man erhält ein Epimerengemisch von 4-[N-(1-Ethoxycarbonyl-3-phenylpropyl)-alanyl]-thiomorpholin-3-carbonsäuren, das mittels "reversed phase" HPLC getrennt wird. Hydrochloride: S,S,R-Form, F. 129; S,S,S-Form, F. 157°.

Analog erhält man als Epimerengemische

4-[N-(1-Ethoxycarbonyl-3-o-tolylpropyl)-alanyl]-thiomorpholin-3-carbonsäure

4-[N-(1-Ethoxycarbonyl-3-p-butylphenylpropyl)-alanyl]-thiomorpholin-3-carbonsäure

4-[N-(1-Ethoxycarbonyl-3-o-methoxyphenylpropyl)-alanyl]-thiomorpholin-3-carbonsäure

4-[N-(1-Ethoxycarbonyl-3-m-methoxyphenylpropyl)-alanyl]-thiomorpholin-3-carbonsäure

4-[N-(1-Ethoxycarbonyl-3-p-methoxyphenylpropyl)-alanyl]-thiomorpholin-3-carbonsäure
4-[N-(1-Ethoxycarbonyl-3-p-butoxyphenylpropyl)-alanyl]-thiomorpholin-3-carbonsäure
4-[N-(1-Ethoxycarbonyl-3-p-chlorphenylpropyl)-alanyl]-thiomorpholin-3-carbonsäure
4-[N-/1-Ethoxycarbonyl-3-p-cyanphenylpropyl)-alanyl]-thiomorpholin-3-carbonsäure
4-[N-(1-Ethoxycarbonyl-3-(3,4-dimethoxyphenyl)-propyl)-alanyl]-thiomorpholin-3-carbonsäure
4-[N-(1-Ethoxycarbonyl-3-(3,4,5-trimethoxyphenyl)-propyl)-alanyl]-thiomorpholin-3-carbonsäure
4-[N-(1-Ethoxycarbonyl-3-(2-chlor-4-methoxyphenyl)-propyl)-alanyl]-thiomorpholin-3-carbonsäure.

Beispiel 8

Analog Beispiel 7 erhält man aus 4-Alanyl-thiomorpholin-3-carbonsäure (S,R-Form) und 2-Oxo-4-phenylbuttersäureethylester in Gegenwart von $NaBH_3CN$ die 4-[N-(1-Ethoxycarbonyl-3-phenylpropyl)-alanyl]-thiomorpholin-3-carbonsäure, Diastereoisomerengemisch aus S,S,R- und R,S,R-Form.

Analog erhält man mit 2-Oxo-4-phenylbuttersäure 4-[N-(1-Carboxy-3-phenylpropyl)-alanyl]-thiomorpholin-3-carbonsäure.

Mit Brenztraubensäure bzw. deren Estern sind analog erhältlich Gemische der S,S,R- und R,S,R-Formen von

4-[N-(1-Carboxy-ethyl)-alanyl]-thiomorpholin-3-carbonsäure
4-[N-(1-Methoxycarbonyl-ethyl)-alanyl]-thiomorpholin-3-carbonsäure
4-[N-(1-Ethoxycarbonyl-ethyl)-alanyl]-thiomorpholin-3-carbonsäure
4-[N-(1-Propoxycarbonyl-ethyl)-alanyl]-thiomorpholin-3-carbonsäure
4-[N-(1-tert.-Butoxycarbonyl-ethyl)-alanyl]-thiomorpholin-3-carbonsäure

4-/N-(1-Hexyloxycarbonyl-ethyl)-alanyl/-thiomorpholin-3-carbonsäure

4-/N-(1-Benzyloxycarbonyl-ethyl)-alanyl/-thiomorpholin-3-carbonsäure.

Beispiel 9

Ein Gemisch von 274 mg 4-Alanyl-morpholin-3-carbonsäure-tert.-butylester (S,S-Form), 206 mg 2-Oxo-4-phenylbuttersäureethylester und 2 g $MgSO_4$ in 10 ml Ethanol wird 16 Stunden bei 20° gerührt. Die so erhaltene Lösung der Schiff-Base wird nach Zugabe von 0,5 g 5%ig. Pd-C 8 Stunden bei 20° und 1 bar bis zum Stillstand hydriert. Man filtriert, dampft ein und erhält ein Diastereoisomerengemisch, aus dem man durch HPLC den 4-/N-(1-Ethoxycarbonyl-3-phenylpropyl)-alanyl/-morpholin-3-carbonsäure-tert.-butylester erhält.

Beispiel 10

Eine Lösung von 0,5 g 4-/N-(1-Carboxy-3-phenylpropyl)-alanyl/-thiomorpholin-3-carbonsäure (S,S,R-Form) in 10 ml Dioxan wird mit einer Lösung von Diazoethan in Dioxan bis zum Bestehenbleiben einer Gelbfärbung versetzt. Man dampft ein und erhält 4-/N-(1-Ethoxycarbonyl-3-phenylpropyl)-alanyl/-thiomorpholin-3-carbonsäure-ethylester, S,S,R-Form. Hydrochlorid, F. 145-146°.

Beispiel 11

Ein Gemisch von 1 g 4-/N-(1-Ethoxycarbonyl-3-phenylpropyl)-alanyl/-thiomorpholin-3-carbonsäure-tert.-butylester (S,S,S-Form), 10 ml Dioxan und 20 ml 4 n HCl in Dioxan wird 2 Tage bei 20° gerührt. Man dampft ein, nimmt mehrfach mit Toluol und Ether auf, dampft erneut ein, löst in Ethylacetat/Acetonitril und fällt mit Ether das 4-/N-(1-Ethoxycarbonyl-3-phenylpropyl)-alanyl/-thiomorpholin-3-carbonsäure-hydrochlorid, S,S,S-Form, F. 157°.

Analog erhält man aus den entsprechenden tert.-Butylestern die Hydrochloride von:

4-/N-(1-Ethoxycarbonyl-3-phenylpropyl)-alanyl/-thiomorpholin-3-carbonsäure-hydrochlorid, S,S,R-Form, F. 129°

4-/N-(1-Ethoxycarbonyl-3-phenylpropyl)-alanyl/-3-methylthiomorpholin-3-carbonsäure

4-/N-(1-Ethoxycarbonyl-3-phenylpropyl)-alanyl/-3-ethylthiomorpholin-3-carbonsäure

4-/N-(1-Ethoxycarbonyl-3-phenylpropyl)-alanyl/-3-propylthiomorpholin-3-carbonsäure

4-/N-(1-Ethoxycarbonyl-3-phenylpropyl)-alanyl/-3-hexylthiomorpholin-3-carbonsäure

4-/N-(1-Ethoxycarbonyl-3-phenylpropyl)-alanyl/-morpholin-3-carbonsäure

4-/N-(1-Ethoxycarbonyl-3-phenylpropyl)-alanyl/-3-methylmorpholin-3-carbonsäure

4-/N-(1-Ethoxycarbonyl-3-(2,5-dimethoxyphenyl)-propyl)-alanyl/-thiomorpholin-3-carbonsäure

4-/N-(1-Ethoxycarbonyl-3-(2,5-dimethoxyphenyl)-propyl)-alanyl/-3-methylthiomorpholin-3-carbonsäure

4-/N-(1-Ethoxycarbonyl-3-(3,4-dimethoxyphenyl)-propyl)-alanyl/-thiomorpholin-3-carbonsäure

4-/N-(1-Ethoxycarbonyl-3-(3,4-dimethoxyphenyl)-propyl)-alanyl/-3-methylthiomorpholin-3-carbonsäure

4-/N-(1-Cyclopentoxycarbonyl-3-phenylpropyl)-alanyl/-thiomorpholin-3-carbonsäure

4-/N-(1-Cyclohexoxycarbonyl-3-phenylpropyl)-alanyl/-thiomorpholin-3-carbonsäure

4-/N-(1-(2-Hydroxyethoxycarbonyl)-3-phenylpropyl)-alanyl/-thiomorpholin-3-carbonsäure

4-/N-(1-(2-Methoxyethoxycarbonyl)-3-phenylpropyl)-alanyl/-thiomorpholin-3-carbonsäure.

Beispiel 12

Ein Gemisch von 2 g 4-/N-(1-Ethoxycarbonyl-3-phenylpropyl)-alanyl7-thiomorpholin-3-carbonsäure-ethylester (S,S,R-Form), 11,5 ml 1 n wässeriger NaOH und 10 ml Ethanol wird 6 Stunden bei 20° gerührt. Man dampft ein, nimmt in Wasser auf, stellt mit HCl auf pH 5 ein, arbeitet wie üblich auf und erhält 4-/N-(1-Carboxy-3-phenylpropyl)-alanyl7-thiomorpholin-3-carbonsäure (S,S,R-Form), F. 159-160°.

Analog erhält man durch Verseifung der entsprechenden S,S,R-Diester die S,S,R-Formen von:

4-/N-(1-Carboxy-3-phenylpropyl)-alanyl7-3-methylthiomorpholin-3-carbonsäure

4-/N-(1-Carboxy-3-phenylpropyl)-alanyl7-3-ethylthiomorpholin-3-carbonsäure

4-/N-(1-Carboxy-3-phenylpropyl)-alanyl7-3-propylthiomorpholin-3-carbonsäure

4-/N-(1-Carboxy-3-phenylpropyl)-alanyl7-3-hexylthiomorpholin-3-carbonsäure

4-/N-(1-Carboxy-3-phenylpropyl)-alanyl7-morpholin-3-carbonsäure

4-/N-(1-Carboxy-3-phenylpropyl)-alanyl7-3-methylmorpholin-3-carbonsäure

4-/N-(1-Carboxy-3-(2,5 ethoxyphenyl)-propyl)-alanyl7-thiomorpholin-3-carbonsäure

4-/N-(1-Carboxy-3-(2,5-dimethoxyphenyl)-propyl)-alanyl7-3-methyl-thiomorpholin-3-carbonsäure

4-/N-(1-Carboxy-3-(3,4-dimethoxyphenyl)-propyl)-alanyl7-thiomorpholin-3-carbonsäure

4-/N-(1-Carboxy-3-(3,4-dimethoxyphenyl)-propyl)-alanyl7-3-methyl-thiomorpholin-3-carbonsäure

4-/$N^2$-(1-Carboxy-3-phenylpropyl)-lysyl7-thiomorpholin-3-carbonsäure

4-/$N^2$-(1-Carboxy-3-phenylpropyl)-lysyl7-3-methyl-thiomorpholin-3-carbonsäure

4-/$N^2$-(1-Carboxy-3-(2,5-dimethoxyphenyl)-propyl)-lysyl7-thiomorpholin-3-carbonsäure

4-/$N^2$-(1-Carboxy-3-(2,5-dimethoxyphenyl)-propyl)-lysyl7-3-methyl-thiomorpholin-3-carbonsäure

4-/$N^2$-(1-Carboxy-3-(3,4-dimethoxyphenyl)-propyl)-lysyl7-thio-morpholin-3-carbonsäure

4-/$N^2$-(1-Carboxy-3-(3,4-dimethoxyphenyl)-propyl)-lysyl7-3-methyl-thiomorpholin-3-carbonsäure.

Beispiel 13

Ein Gemisch von 1g 4-/$N^2$-(1-Ethoxycarbonyl-3-phenylpropyl)-lysyl7-thiomorpholin-3-carbonsäure-tert.-butylester (S,S,R-Form) und 5 ml Trifluoracetanhydrid wird 4 Stunden bei 20° stehengelassen. Nach dem Eindampfen erhält man 4-/$N^2$-(1-Ethoxycarbonyl-3-phenylpropyl)-lysyl7-thiomorpholin-3-carbonsäure, S,S,R-Form.

Beispiel 14

Man hydriert 1 g 4-/N-(1-Ethoxycarbonyl-3-phenylpropyl)-alanyl7-morpholin-3-carbonsäure-benzylester (S,S,S-Form) an 0,5 g 5%igem Pd-C in 15 ml Tetrahydrofuran bei 20° und 1 bar bis zum Stillstand, filtriert, dampft ein und erhält 4-/N-(1-Ethoxycarbonyl-3-phenylpropyl)-alanyl7-morpholin-3-carbonsäure, S,S,S-Form.

Beispiel 15

Analog Beispiel 13 erhält man aus 4-/$N^2$-(1-Ethoxycarbonyl-3-phenylpropyl)-lysyl7-thiomorpholin-3-carbonsäure (S,S,R-Form), durch Verseifung die 4-/$N^2$-(1-Carboxy-3-phenylpropyl)-lysyl7-thiomorpholin-3-carbonsäure (S,S,R-Form).

Beispiel 16

Zu einer Lösung von 332 mg 4-/N-(1-Carboxy-3-phenylpropyl)-alanyl/-thiomorpholin-3-carbonsäure (S,S,R-Form) in 6 ml Ethanol gibt man 0,6 ml 30%iges $H_2O_2$ und rührt 3 Stunden bei 50°. Nach Zugabe weiterer 0,4 ml des Oxydationsmittels rührt man weitere 16 Stunden bei 50°, kühlt ab, arbeitet wie üblich auf und erhält 4-/N-(1-Carboxy-3-phenylpropyl)-alanyl/-thiomorpholin-3-carbonsäure-S-oxid (S,S,R-Form).

Analog erhält man durch Oxydation der entsprechenden Thiomorpholine:

4 -/N-(1-Ethoxycarbonyl-3-phenylpropyl)-alanyl/-thiomorpholin-3-carbonsäure-S-oxid

4-/N-(1-Ethoxycarbonyl-3-phenylpropyl)-alanyl/-thiomorpholin-3-carbonsäure-ethylester-S-oxid

4-/N-(1-Carboxy-3-phenylpropyl)-alanyl/-3-methylthiomorpholin-3-carbonsäure-S-oxid

4-/N-(1-Ethoxycarbonyl-3-phenylpropyl)-alanyl/-3-methylthiomorpholin-3-carbonsäure-S-oxid

4-/N-(1-Ethoxycarbonyl-3-phenylpropyl)-alanyl/-3-methylthiomorpholin-3-carbonsäure-ethylester-S-oxid

4-/N-(1-Cyclopentoxycarbonyl-3-phenylpropyl)-alanyl/-thiomorpholin-3-carbonsäure-S-oxid

4-/N-(1-Cyclohexoxycarbonyl-3-phenylpropyl)-alanyl/-thiomorpholin-3-carbonsäure-S-oxid

4-/N-(1-(2-Hydroxyethoxycarbonyl)-3-phenylpropyl)-alanyl/-thiomorpholin-3-carbonsäure-S-oxid

4-/N-(1-(2-Methoxyethoxycarbonyl)-3-phenylpropyl)-alanyl/-thiomorpholin-3-carbonsäure -S-oxid.

Beispiel 17

Zu einer Lösung von 332 mg 4-/N-(1-Carboxy-3-phenylpropyl)-alanyl7-thiomorpholin-3-carbonsäure (S,S,R-Form) in 2 ml Essigsäure gibt man 1 ml 30%iges $H_2O_2$ und rührt 3 Stunden bei 50°. Nach der üblichen Aufarbeitung erhält man 4-/N-(1-Carboxy-3-phenylpropyl)-alanyl7-thiomorpholin-3-carbonsäure-S,S-dioxid (S,S,R-Form). F.149-151°.

Analog erhält man durch Oxydation der entsprechenden Thiomorpholine:

4-/N-(1-Ethoxycarbonyl-3-phenylpropyl)-alanyl7-thiomorpholin-3-carbonsäure-S,S-dioxid

4-/N-(1-Ethoxycarbonyl-3-phenylpropyl)-alanyl7-thiomorpholin-3-carbonsäure-ethylester-S,S-dioxid (S,S,R-Form),Hydrochlorid, F.208°

4-/N-(1-Carboxy-3-phenylpropyl)-alanyl7-3-methylthiomorpholin-3-carbonsäure-S,S-dioxid

4-/N-1-Ethoxycarbonyl-3-phenylpropyl)-alanyl7-3-methylthiomorpholin-3-carbonsäure-S,S-dioxid

4-/N-1-Ethoxycarbonyl-3-phenylpropyl)-alanyl7-3-methylthiomorpholin-3-carbonsäure-ethylester-S,S-dioxid

4-/N-(1-Cyclopentoxycarbonyl-3-phenylpropyl)-alanyl7-thiomorpholin-3-carbonsäure-S,S-dioxid

4-/N-(1-Cyclohexoxycarbonyl-3-phenylpropyl)-alanyl7-thiomorpholin-3-carbonsäure-S,S-dioxid

4-/N-(1-(2-Hydroxyethoxycarbonyl)-3-phenylpropyl)-alanyl7-thiomorpholin-3-carbonsäure-S,S-dioxid

4-/N-(1-(2-Methoxyethoxycarbonyl)-3-phenylpropyl)-alanyl7-thiomorpholin-3-carbonsäure-S,S-dioxid.

Die nachstehenden Beispiele betreffen pharmazeutische Zubereitungen, die Verbindungen der Formel I oder ihre physiologisch unbedenklichen Salze enthalten:

Beispiel A: Tabletten

Ein Gemisch von 1 kg 1-[N-(1-Ethoxycarbonyl-3-phenylpropyl)-alanyl]-thiomorpholin-3-carbonsäureethylester-hydrochlorid (S,S,R-Form), 4 kg Lactose, 1,2 kg Kartoffelstärke, 0,2 kg Talk und 0,1 kg Magnesiumstearat wird in üblicher Weise zu Tabletten gepreßt, derart, daß jede Tablette 5 mg Wirkstoff enthält.

Beispiel B: Dragees

Analog Beispiel A werden Tabletten gepreßt, die anschließend in üblicher Weise mit einem Überzug aus Saccharose, Kartoffelstärke, Talk, Tragant und Farbstoff überzogen werden.

Beispiel C: Kapseln

Man füllt 1 kg 1-[N-(1-Carboxy-3-phenylpropyl)-alanyl]-thiomorpholin-3-carbonsäure (S,S,R-Form) in üblicher Weise in Hartgelatinekapseln, so daß jede Kapsel 10 mg Wirkstoff enthält.

Beispiel D: Ampullen

Eine Lösung von 1 kg 1-[N-(1-Ethoxycarbonyl-3-phenylpropyl)-alanyl]-thiomorpholin-3-carbonsäureethylester-hydrochlorid (S,S,R-Form) in 30 l zweifach destilliertem Wasser wird steril filtriert, in Ampullen abgefüllt, unter sterilen Bedingungen lyophilisiert und steril verschlossen.
Jede Ampulle enthält 2,5 mg Wirkstoff.

Analog sind Tabletten, Dragees, Kapseln oder Ampullen erhältlich, die einen oder mehrere der übrigen Wirkstoffe der Formel I und/oder ihrer physiologisch unbedenklichen Salze enthalten.

0176903

Merck Patent Gesellschaft
mit beschränkter Haftung

6100 D a r m s t a d t

Patentansprüche:

1. (Thio)morpholine der Formel I

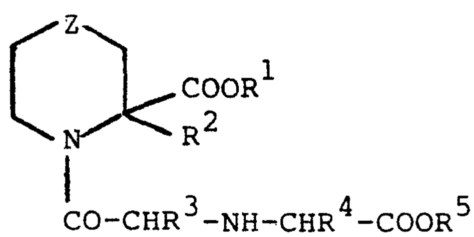

I

worin

Z — O, S, SO oder $SO_2$,

$R^1$ und $R^5$ — gleich oder verschieden sein können und H, Alkyl mit 1 - 4 C-Atomen, Cycloalkyl mit 3 - 7 C-Atomen, Hydroxyalkyl mit 2 - 4 C-Atomen, Alkoxyalkyl mit 2 - 8 C-Atomen oder Benzyl,

$R^2$ — H oder Alkyl mit 1 - 6 C-Atomen,

$R^3$ — $CH_3$ oder $-(CH_2)_4-NH_2$,

$R^4$ — $CH_3$ oder $-(CH_2)_2-Ar$ und

Ar — unsubstituiertes oder ein- oder mehrfach durch Alkyl mit 1 - 4 C-Atomen, Alkoxy mit 1 - 4 C-Atomen, Cl und/oder CN substituiertes Phenyl

bedeuten,

sowie deren Salze.

2. a) 4-[N-(1-Ethoxycarbonyl-3-phenylpropyl)-alanyl]-thiomorpholin-3-carbonsäureethylester.

b) 4-[N-(1-Carboxy-3-phenylpropyl)-alanyl]-thiomorpholin-3-carbonsäure.

3. Verfahren zur Herstellung von Verbindungen der Formel I sowie ihrer Salze, dadurch gekennzeichnet, daß man eine Verbindung der Formel II

E-H II

worin

E für den Rest 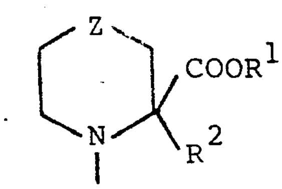 steht und

$R^1$, $R^2$ und Z die in Anspruch 1 angegebene Bedeutung haben

oder eines ihrer reaktionsfähigen Derivate mit einem Dicarbonsäurederivat der Formel III

$$HOOC-CHR^3-NH-CHR^4-COOR^5 \qquad III$$

worin

$R^3$, $R^4$ und $R^5$ die in Anspruch 1 angegebene Bedeutung haben

oder einem seiner reaktionsfähigen Derivate umsetzt, oder daß man eine Verbindung der Formel IV

$$E-CO-CHR^3X \qquad IV$$

mit einer Verbindung der Formel V

$$Y\text{-}CHR^4\text{-}COOR^5 \qquad V$$

worin

| | |
|---|---|
| der eine der Reste X und Y | $NH_2$, |
| der andere | X', |
| X' | Cl, Br, J oder eine reaktionsfähig funktionell abgewandelte OH-Gruppe bedeutet und |
| $R^3$, $R^4$, $R^5$ und E | die in Anspruch 1 bzw. oben angegebenen Bedeutungen haben |

umsetzt

oder daß man eine sonst der Formel I entsprechende Verbindung, die aber an Stelle eines oder mehrerer H-Atome eine oder mehrere Schutzgruppen und/oder reduzierbare oder durch Wasserstoff ersetzbare Gruppe(n) und/oder C-C-Mehrfachbindung(en) enthält, mit solvolysierenden oder reduzierenden Mitteln behandelt und/oder daß man eine Säure der Formel I ($R^1$ und/oder $R^5$ = H) verestert und/oder einen Ester der Formel I ($R^1$ und/oder $R^5$ sind verschieden von H) in die entsprechende Säure der Formel I ($R^1$ und/oder $R^5$ = H) überführt und/oder ein Thiomorpholin der Formel I (Z = S) zum entsprechenden Sulfoxid der

Formel I (Z = SO) oder Sulfon der Formel I (Z = $SO_2$) oder ein Sulfoxid der Formel I (Z = SO) zum entsprechenden Sulfon der Formel I (Z = $SO_2$) oxydiert und/oder eine Verbindung der Formel I durch Behandeln mit einer Säure oder Base in eines ihrer Salze umwandelt.

4. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform bringt.

5. Pharmazeutische Zubereitung, gekennzeichnet durch einen Gehalt an mindestens einer Verbindung der Formel I und/oder einem ihrer physiologisch unbedenklichen Salze.

6. Verbindungen der Formel I und/oder ihre physiologisch unbedenklichen Salze zur Bekämpfung von Krankheiten.

7. Verwendung von Verbindungen der Formel I und/oder ihrer physiologisch unbedenklichen Salze bei der Senkung des Blutdrucks.

8. Verwendung von Verbindungen der Formel I und/oder ihrer physiologisch unbedenklichen Salze zur Herstellung von Arzneimitteln.

Europäisches Patentamt

**EUROPÄISCHER TEILRECHERCHENBERICHT,**
der nach Regel 45 des Europäischen Patent-übereinkommens für das weitere Verfahren als europäischer Recherchenbericht gilt

0176903
Nummer der Anmeldung

EP 85111997.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | GB - A - 2 111 056 (MIDIT SOCIETE FIDUCIAIRE ENREGISTREE)<br>* Zusammenfassung *<br>-- | 1,3-6, 8 | C 07 D 279/12<br>C 07 D 265/30<br>A 61 K 31/535<br>A 61 K 31/54 |
| D,A | EP - A2 - 0 048 159 (UNIVERSITY OF MIAMI)<br>* Ansprüche 1,3 *<br>---- | 1,3-6, 8 | |

| RECHERCHIERTE SACHGEBIETE (Int. Cl 4) |
|---|
| C 07 D 279/00<br>C 07 D 265/00 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-6,8
Unvollständig recherchierte Patentansprüche: -
Nicht recherchierte Patentansprüche: 7
Grund für die Beschränkung der Recherche:

Art. 52(4)EPÜ; Verfahren zur therapeutischen Behandlung des menschlichen oder tierischen Körpers

| Recherchenort | Abschlußdatum der Recherche | Prüfer |
|---|---|---|
| WIEN | 13-01-1986 | BRUS |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument